# EUROPÄISCHE PATENTSCHRIFT

(11) **EP 1 313 064 B1**
(45) Veröffentlichungstag und Bekanntmachung des Hinweises auf die Patenterteilung: **06.07.2005**
(21) Anmeldenummer: 02102587.9
(22) Anmeldetag: 14.11.2002
(51) Int. Cl.: G06T 5/00

(54) **Verfahren und Vorrichtung zur Kalibrierung von und zur Bilderzeugung mit einer magnetempfindlichen Röntgenaufnahmeeinrichtung**
Method and apparatus for calibration of and imaging by means of an X ray imaging apparatus sensitive to magnetic fields
Procédé et dispositif pour la calibration de et la génération d'images par un capteur d'images à rayons X sensible aux champs magnétiques

(30) Priorität: 16.11.2001 DE 10156443
(43) Veröffentlichungstag der Anmeldung: 21.05.2003
(73) Patentinhaber: Philips Intellectual Property & Standards GmbH, 20099 Hamburg (DE); Koninklijke Philips Electronics N.V., 5621 BA Eindhoven (NL)
(72) Erfinder: Sabczynski, Jörg, Dr., Postfach 500442 52088 Aachen (DE); Zylka, Waldemar, Prof. Dr., Postfach 500442 52088 Aachen (DE)
(74) Vertreter: Volmer, Georg, Dipl.-Ing.

(56) Entgegenhaltungen:
- WO-A-98/40847
- DE-A- 19 926 098
- LIU R R ET AL: "SUPER-GLOBAL DISTORTION CORRECTION FOR A ROTATIONAL C-ARM X-RAY IMAGE INTENSIFIER" MEDICAL PHYSICS, AMERICAN INSTITUTE OF PHYSICS. NEW YORK, US, Bd. 26, Nr. 9, September 1999 (1999-09), Seiten 1802-1810, XP000930207 ISSN: 0094-2405

## Beschreibung

Die Erfindung betrifft ein Verfahren und eine Vorrichtung zur Kalibrierung einer magnetfeldempfindlichen Bildaufnahmeeinrichtung sowie ein Verfahren und eine Vorrichtung zur Bilderzeugung mit einer solchen Bildaufnahmeeinrichtung, wie insbesondere einem Bildverstärker in Röntgensystemen, z. B. solchen mit C-Arm. Die Erfindung betrifft ferner ein Röntgensystem mit solchen Vorrichtungen.

Die von Bildverstärkern in Röntgensystemen aufgenommenen Bilder können durch die Einwirkung von äußeren Magnetfeldern, die den Bildverstärker umgeben, verzerrt werden. Dieses Problem zeigt sich in besonderem Maße dann, wenn der Bildverstärker an einem um einen Patienten schwenkbaren Arm (C-Arm) befestigt ist. Es hat sich nämlich überraschend gezeigt, dass die magnetischen Feldstärken in einem Untersuchungsraum lokal sehr unterschiedlich sein können. Dies beruht darauf, dass die zahlreichen elektrischen und metallischen Komponenten von in einem solchen Raum üblicherweise vorhandenen Einrichtungen und Geräten sowie Abschirmungen, die zum Teil aus ferro-magnetischem Material hergestellt sind, das Magnetfeld in ihrer Umgebung erheblich beeinflussen. In diesem Fall wirken sich die äußeren Magnetfelder in jeder Position des Bildverstärkers in anderem Maße auf die Bildverzerrungen aus. Dies gilt aufgrund der in einem Raum stark schwankenden Magnetfelder umso mehr für mobile Röntgensysteme, die in dem Raum verfahren werden können.

Aus der EP 0 479 618 ist ein Verfahren und eine Vorrichtung bekannt, mit dem / der magnetische und geometrische Verzerrungen in einem Röntgenbild entzerrt werden sollen. Zu diesem Zweck wird mit Hilfe eines mit einem Gitter versehenen Testobjektes eine Kalibrierung vorgenommen und eine Kalibrierungstabelle erstellt, mit der jeder Pixelposition in dem Bild die ermittelten Kalibrierungsdaten zugeordnet werden. Die nachfolgend aufgenommenen Bilder werden dann unter Anwendung dieser Tabelle korrigiert. Ähnliche Verfahren und Vorrichtungen mit vergleichbaren Techniken der Ermittlung und Anwendung der Kalibrierungsdaten sind auch aus den Patentanmeldungen DE19926098 (bei wechselnden Einsatzorten) sowie WO9840847 (bei einem einzigen Einsatzort) bekannt.

Eingemeinsamer wesentlicher Nachteil hierbei besteht jedoch darin, dass in dem Fall, in dem sich nach der Kalibrierung das äußere Magnetfeld verändert, vor jeder Aufnahme eine erneute Kalibrierung vorgenommen werden muss. Auch bei einem mobilen Röntgensystem, das an verschiedenen Orten betrieben wird, muss vor jeder Aufnahme eine erneute Kalibrierung durchgeführt werden oder es müssen für jeden denkbaren Einsatzort eine eigene Kalibrierungsdaten ermittelt und gespeichert werden.

Eine Aufgabe, die der Erfindung zugrunde liegt, besteht deshalb darin, ein Verfahren und eine Vorrichtung anzugeben, mit dem / der eine Kalibrierung einer magnetfeldempfindlichen Bildaufnahmeeinrichtung auf relativ einfache und zuverlässige Weise möglich ist.

Weiterhin soll ein Verfahren und eine Vorrichtung angegeben werden, mit dem / der eine solche Kalibrierung in der Weise vorgenommen werden kann, dass sie auch bei einem sich ändernden Magnetfeld nicht wiederholt werden muss.

Mit der Erfindung soll auch ein Verfahren und eine Vomchtung angegeben werden, das / die insbesondere zur Anwendung in mobilen Rontgensystemen zur Kompensation der Einflüsse von Magnetfeldern auf die Bilderzeugung geeignet ist.

Weiterhin soll ein Verfahren und eine Vorrichtung zur Bilderzeugung angegeben werden, das / die insbesondere zur Anwendung mit einer erfindungsgemäß kalibrierten Bildaufnahmeeinrichtung und optional auch zur chirurgischen Navigation ausgestaltet ist.

Schließlich soll auch ein insbesondere für mobile Anwendungen geeignetes Röntgensystem geschaffen werden, bei dem die eingangs genannten Magnetfeldeinflüsse ohne wesentlichen zusätzlichen apparativen Aufwand kompensiert werden können.

Gelöst wird diese Aufgabe gemäß Anspruch 1 mit einem Verfahren zur Kalibrierung einer magnetfeldempfindlichen Röntgen-Bildaufnahmeeinrichtung, das folgende Schritte umfasst: Ermitteln von Kalibrierungsdaten in einer Mehrzahl von gewählten Stellungen der Röntgen-Bildaufnahmeeinrichtung, mit denen ein in der jeweiligen Stellung mit der Röntgen-Bildaufnahmeeinrichtung aufgenommenes Bild entzerrt werden kann; Ermitteln von in den gewählten Stellungen jeweils auf die Röntgen-Bildaufnahmeeinrichtung einwirkenden Magnetfelddaten; und Zuordnen der Magnetfelddaten zu den Kalibrierungsdaten in einer Nachschlagetabelle.

Unter Magnetfeldern sind in diesem Zusammenhang sowohl das Erdmagnetfeld, als auch solche Felder zu verstehen, die durch in der Nähe des Röntgensystems befindliche Vorrichtungen erzeugt oder verändert werden.

Die Aufgabe wird gemäß Anspruch 5 mit einer Vorrichtung zur Durchführung des Kalibrierungsverfahrens mit folgenden Merkmalen gelöst: einem Dreiachsen-Magnetometer zum Ermitteln von auf die Röntgen-Bildaufnahmeeinrichtung einwirkenden Magnetfelddaten; und einer Rechen- und Speichereinheit zum Erstellen und Abspeichern einer Nachschlagetabelle, mit der einer Mehrzahl von ermittelten Magnetfelddaten die zur Entzerrung der dadurch verursachten Bildverzerrungen notwendigen Kalibrierungsdaten zugeordnet sind.

Die Aufgabe wird weiterhin gemäß Anspruch 6 mit einem Verfahren zur Bilderzeugung mit einer erfindungsgemäß kalibrierten Röntgen-Bildaufnahmeeinrichtung gelöst, das folgende Schritte umfasst: Aufnehmen eines Bildes eines Untersuchungsobjektes; Ermitteln von dabei auf die Röntgen-Bildaufnahmeeinrichtung einwirkenden Magnetfelddaten; Vergleichen der ermittelten Magnetfelddaten mit den in der Nachschlagetabelle gespeicherten Magnetfelddaten; Auslesen von gespeicherten Kalibrierungsdaten, die unter zumindest im wesentlichen mit den ermittelten Magnetfelddaten übereinstimmenden Magnetfelddaten gespeichert sind; und Entzerren des aufgenommenen Bildes mit den ausgelesenen Kalibrierungsdaten.

Schließlich wird die Aufgabe gemäß Anspruch 9 mit einer Vorrichtung zur Durchführung des Bilderzeugungsverfahrens mit folgenden Merkmalen gelöst: einem Dreiachsen-Magnetometer zum Ermitteln von auf die Röntgen-Bildaufnahmeeinrichtung einwirkenden Magnetfelddaten; einer Rechen- und Speichereinheit zum Vergleichen der ermittelten Magnetfelddaten mit den in einer Nachschlagetabelle gespeicherten Magnetfelddaten, zum Auslesen von gespeicherten Kalibrierungsdaten, die unter zumindest im wesentlichen mit den ermittelten Magnetfelddaten übereinstimmenden Magnetfelddaten gespeichert sind, und zum Entzerren des aufgenommenen Bildes des Untersuchungsobjektes oder zum Verzerren des Bildes eines in das Untersuchungsobjekt eingeführten Instrumentes mit den ausgelesenen und erforderlichenfalls interpolierten Kalibrierungsdaten, sowie einer Anzeigeeinheit zur Darstellung des entzerrten Bildes des Untersuchungsobjektes oder zur Darstellung des verzerrten Bildes des Untersuchungsobjektes, in das das verzerrte Bild des Instrumentes eingeblendet ist.

Ein wesentlicher Vorteil dieser Lösungen besteht darin, dass zum Zeitpunkt der Kalibrierung noch nicht bekannt sein muss, welche Stärke und Richtung das äußere Magnetfeld bei der späteren Bilderzeugung (d. h. der Untersuchung eines Objektes) aufweisen wird. Die erfindungsgemäße Lösung ist deshalb insbesondere zur dreidimensionalen Bilderzeugung mit Mobilen Röntgensystemen und solchen mit C-Arm, der um ein Untersuchungsobjekt geschwenkt wird, sowie allgemein zur Bilderzeugung mit Röntgensystemen mit Bildverstärkern geeignet. Weiterhin ist die Lösung auch bei der Computer-untetstützten Navigation, mit der bestimmte Stellen in einem Untersuchungsobjektes erreicht werden sollen, vorteilhaft anwendbar.

Schließlich können damit auch Verzerrungen korrigiert werden, die durch eine gekrümmte Oberfläche des Eintrittsfensters des Bildverstärkers entstehen.

Die Unteransprüche haben vorteilhafte Weiterbildungen der Erfindung zum Inhalt.

Die Kalibrierungsdaten können im einfachsten Fall gemäß Anspruch 2 durch Aufnehmen eines Phantomobjektes erfasst oder gemäß Anspruch 3 mit Hilfe eines physikalischen Modells berechnet werden.

Bei den Magnetfelddaten handelt es sich gemäß Anspruch 4 vorzugsweise um die Richtungen sowie die Stärken der Magnetfelder.

Die Ausführung gemäß Anspruch 7 ist schließlich insbesondene für die chirurgische Navigation geeignet.

Weitere Einzelheiten, Merkmale und Vorteile der Erfindung ergeben sich aus der folgenden Beschreibung von bevorzugten Ausführungsformen anhand der Zeichnung. Es zeigt:
- Fig. 1: eine schematische Gesamtansicht eines Röntgensystems mit einer erfindungsgemäßen Vorrichtung und
- Fig. 2: eine schematische Darstellung einer Anordnung zur Kalibrierung des Röntgensystems.

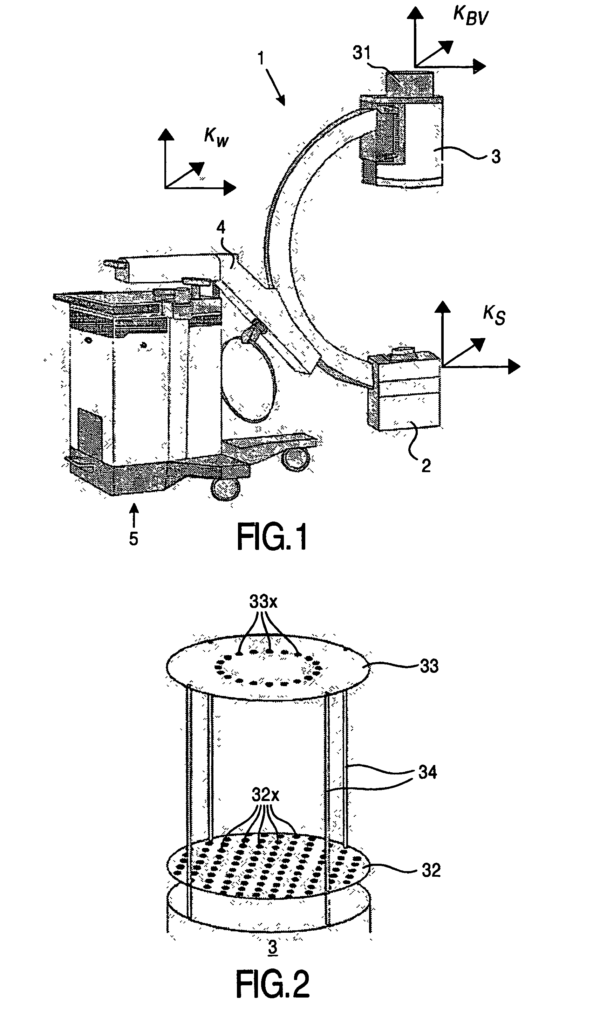

Figur 1 zeigt ein mobiles Röntgensystem mit einem C-Arm 1, an dessen einem Ende eine Röntgenstrahlenquelle 2 und an dessen gegenüberliegendem Ende ein Röntgenstrahlendetektor 3 mit Bildverstärker angeordnet ist. An dem Bildverstärker ist außerdem ein Dreiachsen-Magnetometer 31 befestigt, mit dem die Richtungen und die Stärke des umgebenden Magnetfeldes gemessen werden. Der C-Arm 1 ist schwenkbar an einer Halterung 4 gelagert, die wiederum an einem Tisch 5 defestigt ist. Der Tisch 5 ist fahrbar und mit Bedienungselementen sowie Versorgungs- und Betriebseinrichtungen für das Röntgensystem versehen.

Zwischen die Quelle 2 und den Detektor 3 wird ein Untersuchungsobjekt (Patient) eingebracht, wobei der C-Arm 1 an der Halterung 4 im allgemeinen um mindestens 180 Grad geschwenkt werden kann, um eine optimale Durchleuchtung des zu untersuchenden Bereiches erzielen zu können.

Wie eingangs bereits erwähnt wurde, kann insbesondere der Bildverstärker durch ein äußeres magnetisches Feld gestört oder zumindest so stark beeinflusst werden, dass das aufgenommene Bild verzerrt ist. Aus diesem Grund ist eine Kalibrierung durchzuführen, mit der die von der Stellung des Bildverstärkers abhängigen Magnetfeldstärken kompensiert und somit die dadurch verursachten unterschiedlichen Verzerrungen korrigiert werden.

Die Kalibrierung erfolgt nach der Herstellung des Röntgensystems sowie gegebenenfalls in regelmäßigen Zeitabständen (Serviceintervalle). Die wesentlichen Schritte eines solchen Verfahrens sollen im folgenden erläutert werden. Alternativ dazu sind jedoch auch Abwandlungen dieses Verfahrens oder andere Verfahren anwendbar im Rahmen der angehängten Ansprüche.

Zunächst wird gemäß der Darstellung in Figur 2 ein Phantomobjekt in Form von zwei parallelen Platten 32, 33 an dem Röntgenstrahlendetektor 3 befestigt. Zu diesem Zweck dienen Stangen 34, mit denen die Platten auch parallel gehalten werden. Die erste Platte 32 liegt direkt vor dem Eintrittsfenster des Detektors 3 und ist mit einer Mehrzahl von Kreisflächen oder Kugeln 32x versehen, die auf den Gitterpunkten eines gedachten quadratischen Gitters angeordnet und für Röntgenstrahlen undurchlässig sind. Zwischen der ersten Platte 32 und der Röntgenstrahlenquelle 2 befindet sich die zweite Platte 33 mit einem Abstand von zum Beispiel etwa 37 Zentimeter von der ersten Platte. Die zweite Platte ist mit einer Mehrzahl von genau so großen und für Röntgenstrahlen ebenfalls undurchlässigen Kreisflächen oder Kugeln 33x versehen, die jedoch auf dem Umfang eines zentrierten Kreises angeordnet sind.

Die erste Platte 32 dient zur Ermittlung der Verzenungsparameter eines Projektionsbildes, während die zweite Platte 33 zur Ermittlung der Brennpunktsposition dient, und zwar jeweils für eine Vielzahl von gewählten Schwenkstellungen des C-Arms 1, d.h. Positionen des Bildverstärkers, die zum Beispiel mit Hilfe einer ortsfesten Kamera erfasst werden. Die ermittelten Verzerrungsparameter und Brennpunktspositionen werden als Verzerrungs-Datensätze für jede Position des Bildverstärkers abgespeichert.

Im einzelnen werden die Kreisflächen oder Kugeln 32x der ersten Platte 32 auf den Detektor 3 projiziert, mit einem Segmentierungsalgorithmus erfasst und den einzelnen Kreisflächen oder Kugeln 32x auf der ersten Platte, deren Positionen bekannt sind, zugeordnet. Dadurch sowie durch entsprechende Interpolation zwischen den Flächen können dann die Verzerrungsparameter für "jeden" Röntgenstrahl und somit für jeden Bildpunkt in an sich bekannter Weise berechnet werden.

Weiterhin wird mit Hilfe des durch die zweite Platte 33 auf den Detektor projizierten Bildes des Kreises von Kreisflächen 33x und des Verhältnisses zwischen den Durchmessem dieses Kreises sowie des projizierten Kreises die Brennpunktsposition in an sich bekannter Weise berechnet.

Aus den für jede der Schwenkstellungen des C-Arms 1, d.h. für jede Position des Bildverstärkers, aufgenommenen Verzerrungs-Datensätzen werden Kalibrierungsdaten (Kalibrierungs-Basispunkte) berechnet, die abgespeichert werden und mit denen die durch die Verzerrungen und Brennpunktverschiebungen verursachten Fehler eines in dieser Bildverstärker-Position aufgenommenen Bildes korrigiert (entzent) werden können.

Weiterhin wird mit dem Drei-Achsen-Magnetometer 31 in jeder dieser Positionen des Bildverstärkers das äußere Magnetfeld in der unmittelbaren Umgebung des Bildverstärkers im Hinblick auf seine Richtungen und seine Stärke gemessen. Diese Magnetfelddaten werden zusammen mit den für diese Position ermittelten Kalibrierungsdaten in einander zugeordneter Weise in einer Nachschlagetabelle gespeichert.

Wenn für eine ausreichende Anzahl von Schwenkstellungen des C-Arms 1 (d. h. Positionen des Bildverstärkers) Kalibrierungsdaten sowie Magnetfelddaten ermittelt und in der Nachschlagetabelle abgespeichert wurden, ist die Kalibrierung des Röntgensystems abgeschlossen.

Ergänzend soll hiergbei noch angemerkt werden, dass die Ermittlung der Kalibrierungsdaten auch auf andere Weise, wie zum Beispiel dadurch erfolgen kann, dass anstelle der Bewegung des Bildverstärkers in verschiedene Stellungen ein äußeres Magnetfeld erzeugt wird, das im Hinblick auf seine Richtungen und seine Stärke verändert wird, um für die dadurch verursachten Verzerrungen die Kalibrierungsdaten zu berechnen und unter den jeweiligen Magnetfelddaten in der Nachschlagetabelle abzuspeichem.

Altemativ dazu könnte die Nachschlagetabelle auch auf der Grundlage eines physikalischen Modells der Bildaufnahmeeinrichtung berechnet werden.

Zur Kalibrierung, d.h. zur Erstellung und Abspeicherung der Nachschlagetabelle, ist entweder eine gesonderte Rechen- und Speichereinheit vorgesehen, oder die Kalibrierung wird durch ein entsprechendes Datenverarbeitungsprogramm mit einer in dem betreffenden Röntgensystem vorhandenen Rechnereinheit vorgenommen.

Zur Bilderzeugung bei der Untersuchung eines Patienten oder eines anderen Objektes wird der C-Arm 1 zunächst wie üblich in eine Position geschwenkt, in der der interessierende Bereich durchleuchtet und ein entsprechendes Abbild auf den Bildverstärker projiziert werden kann. Wenn diese Position erreicht ist, wird das Bild in bekannter Weise aufgerommen. Weiterhin wird mit dem Drei-Achsen-Magnetometer 31 das in dieser Position den Bildverstärker umgebende Magnetfeld im Hinblick auf seine Richtungen und seine Stärke erfasst. Diese Magnetfelddaten werden dann mit den entsprechenden Einträgen in der Nachschlagetabelle verglichen. Wenn ein übereinstimmender oder im wesentlichen übereinstimmender Eintrag gefunden wird, werden die diesem Eintrag zugeordneten Kalibrierungsdaten ausgelesen und zur Korrektur des aufgenommenen Bildes in an sich bekannter Weise verwendet.

Wenn keine ausreichende Übereinstimmung zwischen den ermittelten und den in der Tabelle gespeicherten Magnetfelddaten gefunden wird, müssen die betreffenden Kalibrierungsdaten interpoliert werden. Zu diesem Zweck seien die Kalibrierungsdaten als Kalibrierungs-Basispunkte betrachtet. Es sind verschiedene Verfahren anwendbar. Beispielhaft sei hier die Anwendung einer genäherten Delauney Triangulation erläutert.

Es handelt sich dabei deshalb um eine Näherung der Triangulation, weil die Kalibrierungs-Basispunkte auf einer kugelförmigen Fläche liegen, aus Gründen der Einfachheit die durch die Bastspunkte gebildeten Delauney-Dreiecke jedoch als planare Dreiecke behandelt werden sollen. Vor der Interpolation muss deshalb jeder Basispunkt auf solche planaren Dreiecke projiziert werden.

Das ursprüngliche (planare) Verfahren arbeitet wie folgt. Es sei von einem Satz von zu interpolierenden Kalibrierungs-Basispunkten auf einer Kugelfläche ausgegangen. Der Triangulations-Algorithmus führt zu einem Satz von sich nicht schneidenden ebenen Dreiecken, deren Ecken jeweils durch Kalibrierungs-Basispunkte gebildet sind, so dass die gesamte Fläche mit Dreiecken bedeckt ist.

Jeder beliebige Zwischenpunkt P (d.h. ein zu intetpolierender Kalibrierungs-Basispunkt) kann nun eindeutig einem der Dreiecke zugeordnet werden. Die Eckpunkte dieses Dreiecks stellen die drei Kalibrierungs-Basispunkte dar, die dem Punkt P am nächsten liegen. Somit sind die Basispunkte bestimmt, die für die Interpolation zu wählen sind.

Die Delauney Triangulation ist eindeutig. Für eine zweidimensionale Ebene arbeitet der Algorithmus wie folgt: zunächst werden aus dem Satz von Kalibrierungs-Basispunkten alle möglichen Dreiecke gebildet. Diejenigen Dreiecke, deren Eckpunkte kollinear sind, bleiben unberücksichtigt. Wenn der ein Dreieck umschreibende Kreis andere Basispunkte enthält, bleiben das Dreieck ebenfalls unberücksichtigt. Nur wenn diese beiden Fälle nicht gegeben sind, wird das Dreieck verwendet.

Da die Kalibrierungs-Basispunkte jedoch tatsächlich auf einer Kugelfläche liegen, wird zur Vermeidung von geometrischen Verzerrungen und anderen Problemen die Delauney Triangulation an eine sphärische Interpolation angepasst und in der Weise geändert (genähert), dass die Kalibrierungs-Basispunkte in ein dreidimensionales kartesisches Koordinatensystem transformiert werden. Dabei bleibt für jedes Tripel von coplanaren Basispunkten das entsprechende Dreieck unberücksichtigt.

Anstelle des oben genannten umschreibenden Kreises wird eine umschließende Kugel gebildet, und deren Radius wind mit dem dreidimensionalen euklidischen Abstand von jedem anderen Kalibrierungs-Basispunkt verglichen. Man kann nachweisen, dass dieses Kriterium äquivalent zu dem der normalen zweidimensionalen Delauney Triangulation ist, wenn die Basispunkte auf einer idealen Kugelfläche liegen. Wenn die umschließende Kugel andere Basispunkte enthält, bleibt das Dreieck unberücksichtigt. Wenn dies nicht der Fall ist, ist das Dreieck anwendbar.

Zur Vereinfachung sowie zur Beschleunigung der Interpolationsberechnungen wird anstelle des auf der Kugelfläche liegenden Punktes P die Projektion P' des Punktes P auf die ebene Dreiecksfläche betrachtet. Dies führt nur bei großen Dreiecken zu geringfügigen Verzernungseffekten bei den Interpolations-Beiträgen.

Wenn die Triangulation abgeschlossen ist, kann die Interpolation durch die planaren Dreiecke in einfacher Weise unter Anwendung von Schwerpunkts-Koordinaten (baryzentrische Koordinaten) berechnet werden. Ein in der Ebene (C1, C2, C3) liegender Punkt P' kann durch seine baryzentrischen Koordinaten (B1, B2, B3) beschrieben werden. Im folgenden sei angenommen, dass ein Punkt P auf der Kugelfläche, für den die Interpolations-Koeffizienten zu berechnen sind, auf jede zu berücksichtigende planare Dreieckfläche projiziert wird (Punkt P').

Die baryzentrischen Koordinaten beinhalten jeweils eine Information über die relative Position des Punktes P' in Bezug auf eine der Seiten des Dreiecks Für einen Eckpunkt C1 des Dreiecks ist B1 negativ, wenn der Punkt P' jenseits der durch die Eckpunkte C2 und C3 verlaufenden Linie liegt, 0 wenn er auf dieser Linie liegt und positiv, wenn er sich auf der gleichen Seite der Linie befindet, wie der Eckpunkt C1.

Auf diese Weise bieten die baryzentrischen Koordinaten ein einfaches Kriterium für das Auffinden des geeigneten Interpolations-Dreiecks. Nur wenn alle Werte Bi größer als 0 sind, liegt der Punkt P' innerhalb des Dreiecks.

Nachdem nun die baryzentrischen Koordinaten ermittelt wurden, können die für den Punkt P' zu interpolierenden Werte durch eine einfache lineare Kombination ermittelt und somit der interpolierte Kalibrierungs-Basispunkt berechnet werden, mit dem dann das aufgenommene Bild korrigiert bzw. entzerrt wird.

Zur Bilderzeugung und einer gegebenenfalls erforderlichen Interpolation ist wiederum entweder eine gesonderte Rechen- und Speichereinheit oder ein entsprechendes Datenverarbeitungsprogramm vorgesehen, das mit einer in dem betreffenden Röntgensystem vorhandenen Rechnereinheit ausgeführt wird.

Ergänzend sei darauf hingewiesen, dass neben der Bildentzenung eines aufgenommenen Bildes das erfindungsgemäße Prinzip zum Beispiel auch zur chirurgischen Navigation anwendbar ist. Dabei kommt es nicht in erster Linie darauf an, ein aufgenommenes Bild zu entzerren, sondem möglichst genau die Position eines in den Patienten eingeführten Instrumentes (zum Beispiel eines Katheters) zu ermitteln und mit einem entsprechenden Bildverarbeitungssystem in das aufgenommene Bild einzublenden.

Dabei wird einerseits in üblicher Weise ein Röntgenbild des zu untersuchenden Bereiches eines Patienten aufgenommen, ohne dieses zu entzerren. Andererseits wird die aktuelle Position des eingeführten Instruments mit Hilfe eines bekannten Verfahrens oder eines Positionsmessgerätes (zum Beispiel mittels eines kleinen Senders oder einer Induktivität an der Spitze des Instruments) laufend ermittelt. Diese Position wird dann unter (umgekehrter) Anwendung der Nachschlagetabelle, die die Verzerrungseigenschaften des Röntgengerätes beschreibt, verzerrt. Anders ausgedrückt bedeutet dies, dass das (virtuelle) Bild des eingeführten Instruments entsprechend den in Form der Kalibrierungsdaten gespeicherten Abbildungseigenschaften des Röntgengerätes verzerrt wird. Dieses verzerrte Bild wird dann unter Anwendung einer entsprechenden Anzeigeeinheit in das aufgenommene (verzerrte) Röntgenbild eingeblendet, so dass das Instrument nun an der richtigen Stelle in dem Röntgenbild erscheint.

Dies hat den Vorteil, dass auch bei einer laufenden Verfolgung bzw. laufend zu aktualiserenden Darstellung des (im allgemeinen geführten) Instrumentes nur eine Röntgenaufnahme gemacht werden muss Außerdem braucht dabei das Instrument noch nicht eingeführt zu sein, so dass die Röntgenaufnahme dadurch auch nicht beeinträchtigt werden kann.

Eine Entzerrung des Bildes ist dabei nicht notwendig, da nur die tatsächliche Position des Instrumentes relativ zu dem zu untersuchenden Objekt von Bedeutung ist.

## Patentansprüche

1. Verfahren zur Kalibrierung einer magnetfeldempfindlichen Röntgen-Bildaufnahmeeinrichtung mit folgenden Schritten:
- Ermitteln von Kalibrierungsdaten in einer Mehrzahl von gewählten Stellungen der Röntgen-Bildaufnahmeeinrichtung, mit denen ein in der jeweiligen Stellung mit der Röntgen-Bildaufnahmeeinrichtung aufgenommenes Bild entzerrt werden kann;
- Ermitteln von in den gewählten Stellungen jeweils auf die Röntgen-Bildaufnahmeeinrichtung einwirkenden Magnetfelddaten; und
- Zuordnen der Magnetfelddaten zu den Kalibrierungsdaten in einer Nachschlagetabelle.

2. Verfahren nach Anspruch 1,
**dadurch gekennzeichnet,**
**dass** die Kalibrierungsdaten mit Hilfe eines aufgenommenen Phantomobjektes erfasst werden.

3. Verfahren nach Anspruch 1,
**dadurch gekennzeichnet,**
**dass** die Kalibrierungsdaten mit Hilfe eines physikalischen Modells der Bildaufnahmeeinrichtung berechnet werden.

4. Verfahren nach Anspruch 1,
**dadurch gekennzeichnet,**
**dass** die Magnetfelddaten die Richtungen sowie die Stärken der Magnetfelder sind.

5. Vorrichtung zur Durchführung des Verfahrens nach einem der Ansprüche 1 bis 4, mit
- einem Dreiachsen-Magnetometer (31) zum Ermitteln von auf die Röntgen-Bildaufnahmeeinrichtung einwirkenden Magnetfelddaten; und
- einer Rechen- und Speichereinheit zum Erstellen und Abspeichern einer Nachschlagetabelle, mit der einer Mehrzahl von ermittelten Magnetfelddaten die zur Entzerrung der **dadurch** verursachten Bildverzerrungen notwendigen Kalibrierungsdaten zugeordnet sind.

6. Verfahren zur Bilderzeugung mit einer nach einem der Ansprüche 1 bis 4 kalibrierten Röntgen-Bildaufnahmeeinrichtung mit folgenden Schritten:
- Aufnehmen eines Bildes eines Untersuchungsobjektes;
- Ermitteln von dabei auf die Röntgen-Bildaumahmeeinrichtung einwirkenden Magnetfelddaten;
- Vergleichen der ermittelten Magnetfelddaten mit den in der Nachschlagetabelle gespeicherten Magnetfelddaten;
- Auslesen von gespeicherten Kahbrierungsdaten, die unter zumindest im wesentlichen mit den ermittelten Magnetfelddaten übereinstimmenden Magnetfelddaten gespeichert sind; und
- Entzerren des aufgenommenen Bildes mit den ausgelesenen Kalibrierungsdaten.

7. Verfahren zur Bilderzeugung bei der chirurgischen Navigation mit einer Röntgen-Bildaufnahmeeinrichtung, die nach einem der Ansprüche 1 bis 4 kalibriert ist, mit folgenden Schritten:
- Aufnehmen eines Bildes eines Untersuchungsobjektes;
- Ermitteln von dabei auf die Röntgen-Bildaufnahmeeinrichtung einwirkenden Magnetfelddaten;
- Vergleichen der ermittelten Magnetfelddaten mit den in der Nachschlagetabelle gespeicherten Magnetfelddaten;
- Auslesen von gespeicherten Kalibrierungsdaten, die unter zumindest im wesentlichen mit den ermittelten Magnetfelddaten übereinstimmenden Magnetfelddaten gespeichert sind;
- Ermitteln der Position und Aufnehmen eines Bildes eines in das Untersuchungsobjekt eingeführten Instrumentes;
- Berechnen eines verzerrten Bildes des in das Untersuchungsobjekt eingeführten Instrumentes mit Hilfe der ausgelesenen Kalibrierungsdaten; und
- Einblenden des verzerrten Bildes des eingeführten Instrumentes in das aufgenommene Bild des Untersuchungsobjektes.

8. Datenverarbeitungsprogramm mit Programmoode-Mitteln zur Durchführung der Schritte des Verfahrens nach einem der Ansprüche 1 bis 4 oder 6 oder 7, wenn das Programm in einem Computer ausgeführt wird.

9. Vorrichtung zur Durchführung des Verfahrens nach Anspruch 6 oder 7, mit
- einem Dreiachsen-Magnetometer (31) zum Ermitteln von auf die Röntgen-Bildaufnahmeeinrichtung einwirkenden Magnetfelddaten;
- einer Rechen- und Speichereinheit zum Vergleichen der ermittelten Magnetfelddaten mit den in einer Nachschlagetabelle gespeicherten Magnetfelddaten, zum Auslesen von gespeicherten Kalibrierungsdaten, die unter zumindest im wesentlichen mit den ermittelten Magnetfelddaten übereinstimmenden Magnetfelddaten gespeichert sind, und zum Entzerren des aufgenommenen Bildes des Untersuchungsobjektes oder zum Verzerren des Bildes eines in das Untersuchungsobjekt eingeführten Instrumentes mit den ausgelesenen und erforderlichenfalls interpolierten Kalibrierungsdaten, sowie
- einer Anzeigeeinheit zur Darstellung des entzerrten Bildes des Untersuchungsobjektes oder zur Darstellung des verzerrten Bildes des Untersuchungsobjektes, in das das verzerrte Bild des Instrumentes eingeblendet ist.

10. Röntgensystem mit einem Bildverstärker (3) und einer Vorrichtung nach Anspruch 5 oder 9.

## Claims

1. A method for the calibration of an X-ray image pick-up device which is sensitive to magnetic fields, which method includes the following steps:
- determining calibration data in a plurality of selected positions of the X-ray image pick-up device, which calibration data are suitable for distortion correction of an image acquired by the X-ray image pick-up device in the relevant position,
- determining magnetic field data acting on the X-ray image pick-up device in the respective selected positions, and
- associating the magnetic field data with the calibration data in a look-up table.

2. A method as claimed in claim 1, **characterized in that** the calibration data are acquired by means of a recorded phantom object.

3. A method as claimed in claim 1, **characterized in that** the calibration data are calculated by means of a physical model of the image pick-up device.

4. A method as claimed in claim 1, **characterized in that** the magnetic field data represent the directions as well as the strengths of the magnetic fields.

5. A device for carrying out the method as claimed in one of the claims 1 to 4, which device includes
- a triaxial magnetometer (31) for determining magnetic field data acting on the X-ray image pick-up device, and
- an arithmetic and storage unit for forming and storing a look-up table whereby a plurality of magnetic field data determined is associated with the calibration data required for the removal of image distortions caused thereby.

6. A method of imaging, in particular by means of an X-ray image pick-up device calibrated in conformity with one of the claims 1 to 4, which method includes the following steps:
- acquiring an image of the object to be examined,
- determining magnetic field data then acting on the X-ray image pick-up device,
- comparing the magnetic field data thus determined with the magnetic field data stored in the look-up table,
- reading out calibration data stored under magnetic field data which correspond at least essentially to the magnetic field data determined, and
- removing the distortions from the acquired image by means of the calibration data read out.

7. A method of imaging in surgical navigation by means of an X-ray image pick-up device which is calibrated in conformity with one of the claims 1 to 4, which method includes the following steps:
- acquiring an image of an object to be examined,
- determining magnetic field data then acting on the X-ray image pick-up device,
- comparing the magnetic field data thus determined with the magnetic field data stored in the look-up table,
- reading out calibration data stored under magnetic field data which correspond at least essentially to the magnetic field data determined,
- determining the position and acquiring an image of an instrument introduced into the object to be examined,
- calculating a distorted image of the instrument, introduced into the object to be examined, by means of the calibration data read out, and
- reproducing the distorted image of the introduced instrument in the acquired image of the object to be examined.

8. A data processing program which includes program code means for carrying out the steps of the method as claimed in one of the claims 1 to 4 or 6 or 7 when the program is executed in a computer

9. A device for carrying out the method as claimed in claim 6 or 7, which device includes
- a triaxial magnetometer (31) for determining magnetic field data acting on the X-ray image pick-up device,
- an arithmetic and storage unit for comparing the magnetic field data thus determined with the magnetic field data stored in a look-up table, for reading out stored calibration data which are stored under magnetic field data which correspond at least essentially to the magnetic field data determined, and for removing distortions from the acquired image of an object to be examined or for distorting the image of an instrument, introduced into the object to be examined, by way of the read-out and possibly interpolated calibration data, and
- a display unit for displaying the distortion-corrected image of the object to be examined or for displaying the distorted image of the object to be examined in which the distorted image of the instrument is reproduced.

10. An X-ray system which includes an image intensifier (3) and a device as claimed in claim 5 or 9.

## Revendications

1. Procédé de calibrage d'un capteur d'images radiographiques sensible au champ magnétique présentant les étapes suivantes :
- détermination de données de calibrage dans une multitude de positions sélectionnées du capteur d'images radiographiques avec lesquelles une image enregistrée dans la position respective avec le capteur d'images radiographies peut être égalisée;
- détermination de données de champ magnétique agissant respectivement sur le capteur d'images radiographiques dans les positions sélectionnées; et
- attribution des données de champ magnétique aux données de calibrage dans un tableau de référence.

2. Procédé selon la revendication 1,
**caractérisé en ce**
**que** les données de calibrage sont saisies à l'aide d'un objet fantôme enregistré.

3. Procédé selon la revendication 1,
**caractérisé en ce**
**que** les données de calibrage sont calculées à l'aide d'un modèle physique du capteur d'images.

4. Procédé selon la revendication 1,
**caractérisé en ce**
**que** les données des champs magnétiques sont les directions et les intensités des champs magnétiques.

5. Dispositif d'exécution du procédé selon l'une des revendications 1 à 4, avec:
- un magnétomètre triaxial (31) pour la détermmation de données de champ magnétique agissant sur le capteur d'images radiographiques; et
- une unité de calcul et d'enregistrement pour l'élaboration et l'enregistrement d'un tableau de référence avec lequel les données de calibrage nécessaires pour l'égalisation des distorsions d'image ainsi provoquées sont affectées à une multitude de données de champ magnétique déterminées.

6. Procédé de production d'images avec un capteur d'images radiographiques calibré selon l'une des revendications 1 à 4 comportant les étapes suivantes:
- enregistrement d'une image d'un objet d'examen;
- détermination de données de champ magnétique agissant à cette occasion sur le capteur d'images radiographiques;
- comparaison des données de champ magnétique déterminées avec les données de champ magnétique enregistrées dans le tableau de référence;
- lecture des données de calibrage enregistrées qui sont enregistrées parmi les données de champ magnétique correspondant, du moins essentiellement, avec les données de champ magnétique déterminées; et
- égalisation de l'image enregistrée avec les données de calibrage lues.

7. Procédé de production d'image pour la navigation chirurgicale avec un capteur d'images radiographiques qui est calibré selon l'une des revendications 1 à 4, comportant les étapes suivantes:
- enregistrement d'une image d'un objet d'examen;
- détermination de données de champ magnétique agissant à cette occasion sur le capteur d'images radiographiques;
- comparaison des données de champ magnétique déterminées avec les données de champ magnétique enregistrées dans le tableau de référence;
- lecture des données de calibrage enregistrées qui sont enregistrées parmi les données de champ magnétique correspondant, du moins essentiellement, avec les données de champ magnétique déterminées;
- détermination de la position et enregistrement d'une image d'un instrument introduit dans l'objet d'examen;
- calcul d'une image distordue de l'instrument introduit dans l'objet d'examen à l'aide des données de calibrage lues; et
- superposition de l'image distordue de l'instrument introduit dans l'image enregistrée de l'objet d'examen.

8. Programme de traitement de données avec des moyens de programmation pour l'exécution des étapes du procédé selon l'une des revendications 1 à 4, ou 6 ou 7 lorsque le programme est exécuté dans un ordinateur.

9. Dispositif d'exécution du procédé selon l'une des revendications 6 ou 7, avec :
- un magnétomètre triaxial (31) pour la détermination de données de champ magnétique agissant sur le capteur d'images radiographiques; et
- une unité de calcul et d'enregistrement pour la comparaison des données de champ magnétique déterminées avec les données de champ magnétique enregistrées dans un tableau de référence, pour la lecture de données de calibrage enregistrées qui sont enregistrées parmi les données de champ magnétique correspondant, du moins essentiellement, aux données de champ magnétique déterminées et pour l'égalisation de l'image enregistrée de l'objet d'examen ou pour la distorsion de l'image d'un instrument introduit dans l'objet d'examen avec les données de calibrage lues et, si nécessaire, interpolées; et
- une unité d'affichage pour la représentation de l'image égalisée de l'objet d'examen ou pour la représentation de l'image distordue de l'objet d'examen dans laquelle l'image distordue de l'instrument est superposée.

10. Système radiographique avec un amplificateur d'image (3) et un dispositif selon la-revendication 5 ou 9.
